# EUROPÄISCHE PATENTSCHRIFT

(11) **EP 2 165 641 B1**
(45) Veröffentlichungstag und Bekanntmachung des Hinweises auf die Patenterteilung: **13.02.2013**
(21) Anmeldenummer: 09009468.1
(22) Anmeldetag: 22.07.2009
(51) Int. Cl.: A61B 1/005

(54) **Endoskopisches Instrument**
Endoscopic instrument
Instrument endoscopique

(30) Priorität: 17.09.2008 DE 102008047776
(43) Veröffentlichungstag der Anmeldung: 24.03.2010
(73) Patentinhaber: Richard Wolf GmbH, 75438 Knittlingen (DE)
(72) Erfinder: Wehrheim, Frank, 75015 Bretten (DE)
(74) Vertreter: Hemmer, Arnd

(56) Entgegenhaltungen:
- EP-A1- 1 884 185
- WO-A1-2007/057132
- US-A1- 2004 068 161
- US-A1- 2006 258 912

## Beschreibung

Die Erfindung betrifft ein endoskopisches Instrument mit den im Oberbegriff des Anspruchs 1 angegebenen Merkmalen.

Neben endoskopischen Instrumenten mit starren Schäften sind auch Instrumente mit biegsamen Schäften bekannt, welche beispielsweise über Zugseile ausgelenkt werden können. Die in diesen Instrumenten erforderliche Mechanik zum Ablenken bzw. Biegen des Schaftes beansprucht eine gewisse Querschnittsfläche des Schaftes, so dass das nutzbare Lumen im Inneren des Schaftes eingeschränkt wird. Ferner können mit derartigen Schäften nur eingeschränkte Freiheitsgrade bei der Auslenkung realisiert werden.

Darüber hinaus sind Endoskope bekannt, welche im Schaft elektrisch betätigte Aktoren aufweisen, um den Schaft auszulenken. Ein solches Instrument ist beispielsweise aus EP 1 884 185 A1 bekannt. Diese Instrumente sind jedoch relativ kompliziert zu montieren, da sie eine Vielzahl von Einzelteilen und elektrischen Verbindungen im Inneren benötigen.

US 2006/0258912 Al offenbart ein Endoskop mit über elektroaktive Polymere auslenkbaren Abschnitten. Der Aufbau des Endoskopkopfes ist in dieser Druckschrift nicht näher beschrieben.

Im Hinblick auf diesen Stand der Technik ist es Aufgabe der Erfindung, ein endoskopisches Instrument bereitzustellen, welches einen ablenkbaren bzw. biegbaren Schaft aufweist, wobei eine einfache Fertigung des Instrumentes und eine möglichst geringe Einschränkung des nutzbaren Lumens im Inneren des Instrumentes gegeben ist.

Diese Aufgabe wird durch ein endoskopisches Instrument mit den im Anspruch 1 angegebenen Merkmalen gelöst. Bevorzugte Ausführungsformen ergeben sich aus den Unteransprüchen, der nachfolgenden Beschreibung sowie den beigefügten Figuren.

Das erfindungsgemäße endoskopische Instrument, welches für medizinische und technische Zwecke Verwendung finden kann, weist einen Schaft auf, welcher biegbar bzw. flexibel ist. Das heißt, der Schaft kann gegenüber seiner Längsachse im gestreckten Zustand abgewinkelt bzw. gekrümmt werden.

Am distalen Ende des Schaftes ist als Teil des Schaftes ein Endoskopkopf ausgebildet, in welchem die distalseitigen Schnittstellen des Instrumentes angeordnet sind. Die distalseitigen Schnittstellen sind die Austrittsöffnungen der Kanäle, beispielsweise Zugangskanäle für Werkzeug oder Spülkanäle, sowie Fenster von Beleuchtungseinrichtungen und/oder Optiken. Darüber hinaus können diese Schnittstellen auch Fenster bzw. Objektivöffnungen von Kameras und ähnlichen beinhalten. Das heißt, die distalseitigen Schnittstellen sind die Austrittsöffnungen bzw. Endbereiche aller im Inneren des Instrumentenschaftes angeordneten Kanäle, Instrumente und Betrachtungs- und/oder Beleuchtungseinrichtungen.

Erfindungsgemäß bildet der Endoskopkopf einen biegbaren Abschnitt des Schaftes. Das heißt, der distale Endbereich des Schaftes, welcher durch den Endoskopkopf gebildet wird, kann gegenüber der Längsachse des Schaftes abgewinkelt bzw. ausgelenkt werden. Dabei ist vorzugsweise eine Beweglichkeit in allen radialen Richtungen bezüglich der Längsachse des Instrumentes gegeben. Das heißt, der Endoskopkopf kann gegenüber der Längsachse des Schaftes in jede Winkelrichtung zwischen 0 und 360° abgewinkelt werden. Der Endoskopkopf ist erfindungsgemäß einstückig aus einem elektroaktiven Polymer ausgebildet. Das heißt, sowohl die Umfangswandung als insbesondere auch die distalseitige Stirnwandung des Endoskopkopfes sind aus diesem Polymer einstückig ausgebildet. Dabei bildet das elektroaktive Polymer die tragende Struktur des Schaftes im Bereich des Endoskopkopfes. Diese Struktur gibt dem Endoskopkopf die erforderliche mechanische Festigkeit. Es ist denkbar, das Polymer auf der Außenseite mit einer zusätzlichen Schutzschicht für die chemische Aufbereitung des Endoskopes zu versehen. Diese kann beispielsweise als eine auf Adhäsion oder Kohäsion basierende Außenschicht ausgebildet sein. Vorzugsweise wird jedoch kein zusätzlicher Außenschaft auf die Außenseite des Polymers aufgebracht. Auf diese Weise wird die Fertigung und Montage des Endoskopkopfes deutlich vereinfacht, da der Endoskopkopf einstückig aus dem elektroaktiven Polymer gegossen werden kann. Er muss nicht aus Einzelteilen montiert werden. Darüber hinaus muss kein zusätzlicher Außenschaft zum Schutz der Aktoren vorgesehen werden.

Das elektroaktive Polymer bildet gleichzeitig den Aktor, welcher die Auslenkung des Endoskopkopfes bezüglich der Längsachse des Instrumentes bewirkt. Dazu sind in dem Endoskopkopf in dem Polymer Elektroden angeordnet, die mit einer elektrischen Spannung beaufschlagbar sind. Das Anlegen einer Spannung bewirkt eine Größenänderung des Polymeraktors, welche zur Verformung bzw. Auslenkung des Endoskopkopfes genutzt wird. Die Auslenkung wird dadurch erzielt, dass die elektrische Spannung nur an ausgewählte Elektroden oder Elektrodengruppen und nicht jedoch gleichzeitig an alle auf dem Umfang verteilt angeordnete Elektroden angelegt wird. So wird eine Längenänderung des Polymers in Richtung der Längsachse des Schaftes in nur einem bestimmten Umfangsabschnitt erreicht, welcher zu einer Auslenkung des Instrumentes in gewünschter radialer Richtung führt.

Die einstückige Ausbildung aus dem elektroaktiven Polymer ermöglicht eine integrierte Ausbildung des Endoskopkopfes, welcher die distalseitigen Schnittstellen des Instrumentes und den oder die Aktoren zur Auslenkung des Schaftes in einem Bauteil vereint. Da dieses Bauteil einstückig gefertigt, beispielsweise gegossen werden kann, sind keine aufwändigen Montageschritte erforderlich. Darüber hinaus kann dadurch, dass das Polymer selber die Außen- bzw. Umfangswandung des Schaftes im Bereich des Endoskopkopfes bildet, die erforderliche Wandstärke reduziert werden, und so das zur Verfügung stehende Lumen im Inneren des Endoskopkopfes bzw. Schaftes vergrößert werden. Das heißt, durch die Integration der Aktoren in die mechanisch tragende Wandung des Endoskopkopfes kann der erforderliche Bauraum zur Aufnahme der Aktoren reduziert werden, idealerweise ist kein zusätzlicher Bauraum erforderlich.

Bevorzugt sind die Elektroden in das Polymer eingegossen. Die Elektroden sind so ausgebildet, dass sie die Bewegungen des Endoskopkopfes bei dessen Auslenkung mitmachen können. Das Eingießen der Elektroden in das Polymer ermöglicht eine einfache Fertigung, da auf eine Montage der Elektroden an dem Polymer verzichtet werden kann. Vielmehr können die Elektroden in einem Arbeitsgang mit der Ausbildung des integrierten Endoskopkopfes in diesem an den gewünschten Positionen angeordnet werden.

Bevorzugt weisen die Elektroden zumindest eine Elektrodengruppe bestehend aus zumindest einer Steuerelektrode und zumindest einer Referenzelektrode auf. Zwischen der Referenz- und der Steuerelektrode wird die Spannung zur Größenänderung des Polymers angelegt. Referenz- und Steuerelektrode sind in dem elektroaktiven Polymer bevorzugt in axialer Richtung, d. h. in Richtung der Längsachse des Instrumentes voneinander beabstandet, so dass in dieser Richtung vorzugsweise die maximale Längenänderung durch Anlegen der elektrischen Spannung in dem Polymer erreicht werden kann.

Vorzugsweise weist der Endoskopkopf mehrere Elektrodengruppen auf, welche an verschiedenen Winkelpositionen bezüglich der Längsachse des Instrumentes angeordnet sind. Durch diese Anordnung der Elektrodengruppen kann eine Auslenkung des Elektrodenkopfes in verschiedene radiale Richtungen bezüglich der Längsachse erreicht werden. In der Minimalkonfiguration sind zweckmäßigerweise drei gleichmäßig über den Umfang der Längsachse verteilte Elektrodengruppen vorgesehen. Je nach dem, an welche Elektrodengruppen Spannung angelegt wird, kann dabei eine Auslenkung in verschiedene Winkelrichtungen erreicht werden. Durch Erhöhung der Anzahl der Elektrodengruppen welche über den Umfang verteilt angeordnet sind, kann eine feinere Abstufung der Winkel bzw. radialen Richtungen, in welche eine Auslenkung möglich ist, erreicht werden. Im Idealfall kann, indem gegebenenfalls auch mehrere Elektrodengruppen gleichzeitig mit Spannung beaufschlagt werden, eine Auslenkung in beliebiger radialer Richtung bezüglich der Längsachse des Instrumentes erreicht werden. Somit kann eine größtmögliche Beweglichkeit des Instrumentes in beliebigen Richtungen realisiert werden.

Gemäß einer weiteren bevorzugten Ausführungsform kann der Endoskopkopf mehrere Elektrodengruppen aufweisen, welche an verschiedenen axialen Positionen bezüglich der Längsachse des Instrumentes angeordnet sind. Die Elektrodengruppen sind dabei vorzugsweise so ausgebildet, dass sie unabhängig voneinander mit Spannung beaufschlagt werden können, so dass eine Auslenkung bzw. Abwinklung des Schaftes an unterschiedlichen axialen Positionen realisiert werden kann. Dadurch lassen sich unterschiedliche Krümmungswinkel bei der Auslenkung des Elektrodenkopfes und auch unterschiedliche Auslenkungsrichtungen über die axiale Länge des Endoskopkopfes erreichen, so dass eine sehr große Beweglichkeit des Endoskopkopfes in verschiedene Richtung möglich wird. Im Extremfall kann der Elektrodenkopf in einer dreidimensionalen Schlangenbewegung geführt bzw. ausgelenkt werden.

Die einzelnen Elektrodengruppen können jeweils aus einer Mehrzahl einzelner Elektroden bestehen, welche gegebenenfalls gleichzeitig mit Spannung beaufschlagt werden können. So können Einzelaktoren, welche jeweils aus zwei Elektroden bestehen in einer Elektrodengruppe gestapelt werden, so dass die Elektrodengruppe eine Aktorgruppe bildet. Die einzelnen Elektroden sind nur sehr gering voneinander beabstandet, so dass die Größenänderungen zwischen zwei Elektroden sehr klein sind. Durch die Stapelung derartiger Aktoren in einer Richtung und eine entsprechende Anordnung einer Vielzahl von Elektroden hintereinander, können in dem Polymer größere Form- bzw. Größenänderungen realisiert werden.

Bevorzugt ist zumindest eine Elektrodengruppe vorgesehen, welche mehrere in axialer Richtung bezüglich der Längsachse des Instrumentes voneinander beabstandete Steuerelektroden aufweist, welche vorzugsweise unabhängig voneinander mit Spannung beaufschlagbar sind. Durch die unabhängige Beaufschlagung der einzelnen Elektroden mit Spannung kann die Beweglichkeit des Instrumentes erhöht werden, d. h. die Verformung in einzelnen Winkelbereichen und auch in axialer Richtung kann sehr flexibel eingestellt werden, so dass eine große Beweglichkeit des Schaftes erreicht wird. Wie vorangehend beschrieben können jedoch auch mehrere Steuerelektroden gleichzeitig an Spannung gelegt werden und beispielsweise fest zu elektrisch miteinander verbundenen Elektrodengruppen zusammengefasst werden, um größere Formänderungsbereiche zu schaffen.

Die Elektrodengruppen weisen vorzugsweise jeweils eine Vielzahl von Steuerelektroden und eine Vielzahl von Referenzelektroden auf, welche in axialer Richtung einander abwechselnd angeordnet sind. Das heißt, es folgt immer eine Steuerelektrode auf eine Referenzelektrode, die durch eine anliegende Spannung Größen- bzw. Formänderung des Polymers bewirken. Das Polymer bildet dabei zwischen den Elektroden vorzugsweise ein Dielektrikum. Derartige Elektrodengruppen, welche gemeinsam mit dem Polymer, in welches sie eingebettet sind, eine Aktorgruppe bilden, sind vorzugsweise verteilt über den Umfang des Schaftes angeordnet, so dass je nach dem welche Elektrodengruppen mit Spannung beaufschlagt werden eine Auslenkung des Schaftes in unterschiedlichen Winkelrichtungen bezüglich der Längsachse möglich ist.

Die Steuer- und/oder Referenzelektroden können dabei so ausgebildet sein, das sie einzeln mit Spannung beaufschlagt werden können. Die können jedoch auch zu Gruppen zusammengefasst und elektrisch miteinander verbunden sein. Bevorzugt sind dazu die Vielzahl der Steuerelektroden und die Vielzahl von Referenzelektroden jeweils als Kammstruktur ausgebildet und die beiden Kammstrukturen greifen mit ihren Zähnen ineinander ein. So kommt immer eine Referenzelektrode zwischen zwei Steuerelektroden zu liegen. Die einzelnen Elektroden jeder Kammstruktur sind dabei bevorzugt an einer Seite, d. h. einem Ende elektrisch leitend miteinander verbunden. Diese Seite bildet die geschlossene Seite des Kammes. Von der entgegengesetzten Seite her greifen dann die jeweils anderen Elektroden in die Freiräume zwischen den Elektroden ein.

Gemäß einer weiteren bevorzugten Ausführungsform kann die Referenzelektrode wendel- bzw. schraubenförmig ausgebildet sein und es können Steuerelektroden zwischen einzelnen Schraubengängen der Referenzelektroden angeordnet sein. Die Steuerelektroden können dabei ebenfalls schraubenförmig bzw. wendelförmig ausgebildet sein. Alternativ ist es auch möglich einzelne segmentförmige Steuerelektroden zwischen den Gewindegängen der Referenzelektrode anzuordnen und die Steuerelektroden so auszubilden, das sie einzeln oder in einzelnen Gruppen mit Spannung beaufschlagbar sind. Auf diese Weise ist es sehr einfach möglich, mit einer gemeinsamen durchgehenden Referenzelektrode mehrere Elektroden- bzw. Aktorgruppen zu bilden, welche einzeln durch Anlegen einer elektrischen Spannung betätigt bzw. aktiviert werden können. Auf diese Weise lässt sich relativ platzsparend in der Wandung des Endoskopkopfes eine Vielzahl von Elektroden unterbringen, welche individuell oder in Gruppen mit Spannung beaufschlagt werden können, um eine große Beweglichkeit des Endoskopkopfes in verschiedene Richtungen realisieren zu können.

So kann auch eine Mehrzahl von Elektrodengruppen eine gemeinsame Referenzelektrode, insbesondere in schraubenförmiger Gestalt aufweisen. Auf diese Weise werden die erforderlichen elektrischen Verbindungen, welche in dem Polymer angeordnet werden müssen vereinfacht, da nicht eine Vielzahl von Referenzelektroden elektrisch miteinander verbunden werden müssen, sondern stattdessen eine durchgehende schraubenförmige Referenzelektrode für mehrere Elektrodengruppen verwendet werden kann. Eine derartige schraubenförmige Leiterstruktur lässt sich relativ einfach ausbilden und in das elektroaktive Polymer einbetten.

Die vorangehend beschriebene Ausgestaltung der Elektroden- bzw. Akoranordnungen ist nicht auf den beschriebenen Endoskopkopf beschränkt. Vielmehr können in dem Schaft auch proximalseitig des Endoskopkopfes weitere biegbare bzw. auslenkbare Abschnitte aus einem elektroaktiven Polymer mit darin angeordneten Elektroden ausgebildet sein. Bevorzugt weist der Schaft über seine gesamte Länge derart biegbare Abschnitte auf, so dass ein sehr beweglicher Schaft gebildet wird, welcher sich auch durch gewundene bzw. geschlängelte Körperkanäle bewegen kann. Auch bei diesen biegbaren Abschnitten bildet das elektroaktive Polymer vorzugsweise die tragende Wandung bzw. tragende Struktur des Schaftes, d. h. das elektroaktive Polymer erstreckt sich vorzugsweise im Wesentlichen über die gesamte Wandstärke des Schaftes. Gegebenenfalls kann am Außenumfang eine Beschichtung in der oben anhand des Endoskopkopfes beschriebenen Weise angeordnet sein. Die Anordnung der Elektroden und deren Funktion entspricht auch bei derartig biegbaren proximalseitigen Schaftabschnitten den vorangehend anhand des Endoskopkopfes beschriebenen Ausgestaltungen.

Zweckmäßigerweise sind die Elektroden mit Steuerleitungen verbunden, welche sich zum proximalen Ende des Schaftes erstrecken. Dort können sie dann mit einer Steuereinrichtung, welche die einzelnen Aktoren ansteuert, verbunden sein. Die Steuerleitungen sind vorzugsweise als metallische Leiter ausgebildet, welche in das Wandungsmaterial des Schaftes, welches vorzugsweise aus einem Kunststoff, besonders bevorzugt ebenfalls aus einem elektroaktiven Polymer besteht, eingebettet sind. Sie können jedoch auch an der Innen- oder Außenseite der Schaftwandung angebracht bzw. aufgebracht sein. In biegbaren bzw. beweglichen Abschnitten des Schaftes sind diese Steuerleitungen ebenfalls vorzugsweise biegbar. In starren Abschnitten können sie ebenfalls starr ausgebildet sein. Die Einbettung in einem Kunststoff hat den Vorteil, dass durch diesen gleichzeitig die elektrische Isolation gewährleistet wird. Ferner wird die Montage vereinfacht, da die elektrischen Leiter bzw. Steuerleitungen beim Gießen des Kunststoffes in die Form des Schaftes gleich in diesen eingebettet werden können.

Gemäß einer weiteren bevorzugten Ausführungsform weist der Endoskopkopf zumindest einen am distalen Ende angeordneten Kraftsensor auf. Dabei ist der Kraftsensor vorzugsweise so orientiert, das er zumindest eine in Richtung der Längsachse des Schaftes, wirkende Kraftkomponente erfassen kann. Bevorzugt ist eine Mehrzahl von Kraftsensoren am distalen Ende, insbesondere an der Stirnfläche des distalen Endes angeordnet. Die Kraftsensoren sind dabei vorteilhafterweise über den Umfang verteilt nahe dem Außenumfang angeordnet. Besonders bevorzugt sind die Kraftsensoren mit ihrer Hauptwirkungsrichtung geneigt z. B. in einer Richtung von 45° zur Längsachse des Instrumentes bzw. zur Vorschubrichtung des Instrumentes orientiert. So können sie Kraftkomponenten, welche in Umfangsrichtung auf den Schaft wirken und Kraftkomponenten, welche in Längsrichtung des Schaftes wirken, erfassen. Die Kraftsensoren dienen dazu, ein Anstoßen des distalen Endes des Endoskopes an Gewebeteile schnell erfassen zu können, um eine Beschädigung von Gewebe beim Vorschub zu verhindern. So können die Kraftsensoren, wie nachfolgend beschrieben werden wird, mit einer Steuereinrichtung zusammenwirken, welche die Auslenkung bzw. Biegung des Endoskopkopfes steuert.

Durch die Anordnung einer Vielzahl von Kraftsensoren verteilt über den Umfang der distalseitigen Stirnfläche kann genau erfasst werden an welchem Umfangswinkel das Endoskop anstößt, so dass es in entgegengesetzter Richtung abgelenkt und weiter vorgeschoben werden kann.

Der oder die Kraftsensoren können beispielsweise als kapazitive Kraftsensoren ausgebildet sein. Zweckmäßigerweise sind die Kraftsensoren direkt in das elektroaktive Polymer eingebettet bzw. eingegossen. Im Idealfall sind hierzu lediglich Elektroden erforderlich. Die Kapazitätsänderung zwischen den Elektroden erfolgt durch die elastische Stauchung des Polymers zwischen den Elektroden des Sensors.

Vorzugsweise ist eine Steuereinrichtung vorgesehen, welche mit dem oder den Kraftsensoren derart verbunden ist, dass sie von den Kraftsensoren Signale, welche repräsentativ für die jeweils erfasste Kraft sind, empfängt. Die Verbindung erfolgt bevorzugt in elektrischer Weise, d. h. von den Kraftsensoren werden elektrische Signale erzeugt, welche zu der Steuereinrichtung übertragen werden. Die Steuereinrichtung wertet die erfassten Kraftsignale aus.

Darüber hinaus ist die Steuereinrichtung derart ausgebildet, dass sie die Aktoren zur Auslenkung des Schaftes steuert. Das heißt, die Steuereinrichtung veranlasst die Aktoren, den Schaft in bestimmter Weise auszulenken. Dabei ist die Steuereinrichtung so ausgebildet, dass die Aktoren von der Steuereinrichtung unter Berücksichtung der von dem Kraftsensor erfassten Kraft gesteuert werden. Dies ermöglicht eine automatische oder teilweise automatische Steuerung der Auslenkung des Endoskopkopfes in Abhängigkeit der auf die Stirnseite wirkenden Kräfte. So kann die Steuereinrichtung in dem Fall, dass von dem Kraftsensor eine Kraft, welche auf die distale Stirnseite des Endoskopes wirkt, erfasst wird, den Schaft bzw. den Endoskopkopf durch Steuerung der Aktoren ablenken, um einen weiteren Vorschub in Richtung der wirkenden Kraft zu verhindern. Auf diese Weise kann eine Verletzung von Gefäßen beim Vorschub des Instrumentes verhindert werden, da von der Steuereinrichtung ein automatisches Ausweichen von Hindernissen ermöglicht wird. Eine Kraft wirkt auf den Kraftsensor dann, wenn sich ein Hindernis dem Endoskop bei dessen Vorschub entgegenstellt. Durch entsprechende Auslenkung des Schaftes kann diesem Hindernis ausgewichen werden. Gleichzeitig ist es möglich, dass von der Steuereinrichtung der Bedienperson das entsprechende Hindernis signalisiert wird. Dies kann beispielsweise optisch, akustisch oder auch haptisch erfolgen.

Bevorzugt sind am distalen Ende des Schaftes mehrere Kraftsensoren am stirnseitigen Randbereich angeordnet, vorzugsweise derart, dass eine auf das distale Ende wirkende Kraft richtungsabhängig bestimmbar ist. Die Anordnung am distalen Randbereich, d. h. am Außenumfang der distalen Stirnfläche hat den Vorteil, dass gerade auch Hindernisse welche nur vom Randbereich des distalen Endes berührt werden, über die Kraftsensoren detektiert werden können. In dem Fall, dass mehrere Kraftsensoren über den Umfang der distalen Stirnfläche verteilt angeordnet werden, ist darüber hinaus erfassbar, an welchem Umfangsbereich sich das Hindernis dem Instrument entgegenstellt. Entsprechend kann die Steuereinrichtung reagieren und die Aktoren so steuern, dass der Schaft in eine abgewandte Richtung ausgelenkt wird, um dem Hindernis auszuweichen. So kann über die mehreren Kraftsensoren bestimmt werden, aus welcher Richtung die Kraft kommt, d.h. in welcher Richtung sich das Hindernis dem Vorschub in den Weg stellt. Besonders bevorzugt sind die Kraftsensoren am Randbereich des distalen Endes so angeordnet, dass sie nicht nur eine Kraft in axialer Richtung bezogen auf die Längsachse des gestreckten Instrumentenschaftes erfassen, sondern auch Kraftkomponenten, welche in radialer Richtung auf das Ende einwirken. So können die Kraftsensoren im Winkel zur Stirnfläche und zur Längsachse angeordnet werden, beispielsweise 45°, sodass ihre Hauptwirkungsrichtung zur Längsachse und zur Stirnfläche geneigt ist. Auf diese Weise können noch besser Hindernisse erfasst werden, welche am Randbereich der Stirnseite sich dem Vorschub des Instrumentes in den Weg stellen.

Weiter ist es bevorzugt, dass die Steuereinrichtung mit Bedienelementen verbunden ist, über welche die Auslenkung des Schaftes einstellbar ist. Dies ermöglicht eine semi-manuelle Bewegungssteuerung. Über die Bedienelemente kann ein Benutzer des Instrumentes die Vorschubrichtung und damit die erforderliche Auslenkung des Endoskopkopfes vorwählen. Dies kann beispielsweise auf Grundlage eines von der Endoskopoptik erzeugten Bildes erfolgen. Die Steuereinrichtung steuert dann die Aktoren so, dass der Schaft des Instrumentes, d.h. insbesondere dessen distales Ende bzw. der Endoskopkopf in entsprechender Weise von den Aktoren ausgelenkt bzw. abgewinkelt wird. Gleichzeitig ist die Steuereinrichtung jedoch auch mit den Kraftsensoren verbunden und kann eine automatische Auslenkung veranlassen, wenn die Kraftsensoren einen Widerstand erfassen. Dabei kann die Steuereinrichtung so ausgebildet sein, dass die automatische Auslenkung der manuellen Auswahl der Auslenkung vorrangig überlagert wird, sodass ein Umfahren von Hindernissen bzw. ein Ausweichen in jedem Fall automatisch erfolgt, unabhängig davon, welche Auslenkung eine Bedienperson manuell ausgewählt hat.

Alternativ ist es jedoch auch möglich, die Steuereinrichtung derart auszubilden, dass die Aktoren in Abhängigkeit von dem zumindest einem Kraftsensor erfassten Kraft automatisch gesteuert werden. Auf diese Weise kann die Steuereinrichtung eine vollautomatische Auslenkung beim Vorschub erreichen. So kann beim Vorschub das Instrument einen vorgegebenen Kanal, beispielsweise einer Körperöffnung selbsttätig folgen, ohne das eine Bedienperson auf richtige Auslenkung des Instrumentenschaftes zu achten hat. Es ist zu verstehen, dass die Steuereinrichtung auch derart ausgebildet sein kann, dass sie wahlweise beide Modi zulässt, den beschriebenen vollautomatischen Modus oder den zuvor beschriebenen semi-manuellen Modus.

Besonders bevorzugt ist die Steuereinrichtung derart ausgebildet, dass bei Erfassung einer Druckkraft oberhalb eines vorbestimmten Grenzwertes an einem Kraftsensor der Schaft durch Steuerung der Aktoren in einer der erfassten Kraft entgegengesetzten Richtung ausgelenkt wird. Das die Auslenkung erst oberhalb eines bestimmten Grenzwertes er-' folgt, hat den Sinn, dass geringe Widerstände, welche sich durch Reibung beim Vorschub des Instrumentes ergeben, ignoriert werden können und nur solche Kräfte von der Steuereinrichtung berücksichtigt werden, welche tatsächlich auf ein Hindernis schließen lassen. Wie oben beschrieben, ist es bevorzugt, mehrere Kraftsensoren an der distalen Stirnseite des Schaftes anzuordnen, welche auftretende Kräfte richtungsabhängig erfassen. Auf Grundlage der richtungsabhängigen Krafterfassung ist es der Steuereinrichtung dann möglich, die Aktoren so zu steuern, dass der Schaft in eine entgegengesetzte Richtung der auftretenden Kraft ausgelenkt wird. Das heißt, wenn beispielsweise ein Kraftsensor ein Hindernis am Rand der Stirnseite des Instrumentes in einer Position von 0° erfasst, wird der Schaft dann durch Betätigung der Aktoren durch die Steuereinrichtung vorzugsweise in die Richtung von 180° ausgelenkt. Auf diese Weise wird ein Hindernis sicher umfahren.

Weiter bevorzugt ist die Steuereinrichtung derart ausgebildet, dass bei Erfassung einer Druckkraft oberhalb eines vorbestimmten Grenzwertes an mehreren Kraftsensoren der axiale Vorschub des Schaftes gestoppt und/oder ein Warnsignal ausgegeben wird. Die Erfassung an mehreren Kraftsensoren lässt auf eine Kraft schließen, welche stirnseitig in axialer Richtung auf die Instrumentenstirnseite wirkt. In diesem Fall trifft das Instrument stumpf auf ein Hindernis. Im Falle einer solchen stumpfen Kollisionen, z. B. in einer Sackgasse, kann eine automatische Auslenkung nicht mehr erfolgen. In diesem Fall soll die Steuereinrichtung dann dem Bediener ein Warnsignal ausgeben, sodass er den Vorschub des Instrumentes stoppen kann. Im Falle eines automatisierten Vorschubes kann der Vorschub in einem solchen Fall auch automatisch gestoppt werden. Das Warnsignal kann beispielsweise auch optisch, haptisch oder akustisch sein.

Gemäß einer weiteren bevorzugten Ausführungsform sind über die axiale Länge des Schaftes verteilt, mehrere unabhängig voneinander von der Steuereinrichtung betätigte Aktoren angeordnet. Diese Ausgestaltung ermöglicht eine Bewegbarkeit des Schaftes über eine größere axiale Länge. Durch die unabhängige Ansteuerbarkeit der Aktoren ist es dabei möglich, den Schaft in unterschiedlichen axialen Bereichen in unterschiedliche Richtungen auszulenken. So kann der Schaft auch schlangenlinienförmig bewegt werden. Es wird somit möglich, mit dem Schaft auch mehrfach gekrümmten Kanälen zu folgen.

Insbesondere ist es dabei bevorzugt, dass die Steuereinrichtung so ausgebildet ist, dass sie beim Vorschub des Schaftes die proximal gelegenen Aktoren so steuert, dass der Schaft der, von den distal gelegenen Aktoren verursachten Auslenkung, nachgeführt wird. Das heißt, beim Vorschub werden die proximal gelegenen Aktoren so betätigt, dass sich die Krümmung der distalen Aktoren proportional der Vorschubbewegung des Schaftes auf die Aktoren in proximaler Richtung überträgt. So kann der Schaft beim Vorschub einem gekrümmten Kanalverlauf folgen. Insbesondere wird es möglich, einem mehrfach gekrümmten Kanalverlauf auch in Schlangenlinien zu folgen. In umgekehrter Richtung wird beim Herausziehen des Instrumentes aus dem Kanal die Steuereinrichtung die weiter distalwärts gelegenen Aktoren so steuern, dass sie der Krümmungfolgen. Eine solche Steuerung erfolgt vorzugsweise automatisch.

Dazu ist vorzugsweise eine automatische Vorschubeinrichtung und/oder eine Erfassungseinrichtung zum Erfassen des Vorschubes vorhanden, welche mit der Steuereinrichtung zusammenwirken. Das heißt, im Fall eines manuellen Vorschubs der Vorschubweg erfasst und der Steuereinrichtung als Eingangsgröße zugeleitet, sodass die Steuereinrichtung bei der axialen Bewegung die Aktoren so steuern kann, dass der Instrumentenschaft einem gekrümmten Kanalverlauf folgt. Alternativ kann der gesamte Vorschub automatisch gesteuert werden, d. h. die Steuereinrichtung über einen Antrieb auch den Vorschub bewirkt. Dabei können jedoch Eingabemittel bzw. Bedienelemente vorgesehen sein, über welchen der Nutzer den gewünschten Vorschub wählen kann. Das heißt, hier wird der Vorschub dann nicht direkt mechanisch durch den Nutzer veranlasst, sondern indirekt über die zwischengeschaltete Steuereinrichtung. Diese Ausgestaltung hat den Vorteil, dass die Steuereinrichtung, wie oben beschrieben, im Falle einer stumpfen Kollision am distalen Schaftende den Vorschub selbsttätig stoppen kann.

Die Kraftsensoren sind vorzugsweise in ein Kunststoffmaterial, aus welchem das distale Ende des Schaftes gefertigt ist, eingebettet. So kann man die Kraftsensoren in das distale Ende, insbesondere in den Endoskopkopf integrieren. Zum einen vereinfacht sich dadurch die Fertigung. Zum anderen wird der Raumbedarf der Sensoren sehr klein gehalten, sodass das nutzbare Lumen im Inneren des Schaftes nicht wesentlich beeinträchtigt wird, und vorzugsweise die gesamte distale Stirnseite des Schaftes für die distalen Schnittstellen, d.h. Austrittsöffnung der Kanäle im Schaft und optischen Elemente bzw. Beleuchtungseinrichtungen zur Verfügung steht. Die Kraftsensoren sind vorzugsweise in das Kunststoffmaterial eingegossen. Dabei kann das Kunststoffmaterial selbst einen Teil des Kraftsensors bilden. Beispielsweise können Elektroden beabstandet voneinander in das Kunststoffmaterial eingegossen werden. Bei Krafteinwirkung verformt sich das Kunststoffmaterial zwischen den Elektroden, wodurch sich elektrischer Widerstand oder die Kapazität zwischen den Elektroden verändert, was erfassbar ist. In Kenntnis der mechanischen Eigenschaften, insbesondere des Elastizitätsmoduls, kann dann auf die einwirkende Kraft geschlossen werden kann. Auf diese Weise können die Kraftsensoren sehr einfach in dem Kunststoffmaterial des Schaftes ausgebildet werden.

In ähnlicher Weise kann auch die erfolgte Auslenkung bzw. Abwinklung des Endoskopschaftes in der Weise erfasst werden, dass sich durch die Verformung des elektroaktiven Polymers zwischen zwei Elektroden der elektrische Widerstand ändert, was direkt zwischen den Elektroden erfasst werden kann. Das heißt, hier sind keine zusätzliche Sensoren in der Schaftwandung bzw. Wandung des Endoskopkopfes anzuordnen, vielmehr muss lediglich der Widerstand zwischen den für die Betätigung der Aktoren verwendeten Elektroden erfasst werden.

Das endoskopische Instrument gemäß der Erfindung weist zweckmäßigerweise eine elektronische Steuereinrichtung auf, welche mit den Elektroden verbunden ist und die Verformung bzw. Auslenkung des Schaftes steuert. Das heißt, die Steuereinrichtung beaufschlagt die erforderlichen Elektroden mit Spannung um eine gewünschte Bewegungs- oder Auslenkungsrichtung des Schaftes oder Endoskopkopfes zu gewährleisten. Die Auslenkungsrichtung kann dabei im semi-manuellen Betrieb zusätzlich über geeignete Bedienelemente von dem Nutzer des Instrumentes vorgegeben werden.

Die Erfindung ermöglicht ein besonderes Verfahren zur Steuerung eines endoskopischen Instrumentes, welches in der vorangehend beschriebenen Weise ausgebildet ist. Gemäß diesem Verfahren, werden, wie bereits oben anhand der Vorrichtung beschrieben, beim Vorschub des Instrumentes, die auf das distale Ende des Schaftes wirkenden Kräfte erfasst und eine Auslenkung des beweglichen Schaftes in Abhängigkeit der erfassten Kräfte veranlasst. Auf diese Weise können über die Krafterkennung Hindernisse erkannt, und durch entsprechende Auslenkung des Schaftes umfahren werden. Diesbezüglich wird auf die obige Beschreibung verwiesen.

Wie bereits oben beschrieben, ist es besonders bevorzugt, dass die Auslenkung des Schaftes jeweils in eine der erfassten Kraft abgewandte Richtung erfolgt. Auf diese Weise werden Hindernisse, welche nur im Randbereich oder Teilbereichen der distalen Stirnfläche des Schaftes gelegen sind umfahren.

In dem Fall, dass eine in axialer Richtung auf das distale Ende wirkende Kraft erfasst wird, wird der Vorschub des Instrumentes vorzugsweise gestoppt und/oder ein Warnsignal ausgegeben, sodass der Bediener des Instrumentes den Vorschub stoppen kann.

Hinsichtlich der näheren Ausgestaltung des Verfahrens wird auf die obige Beschreibung der Vorrichtung verwiesen, aus welche sich auch die entsprechenden Merkmale des Verfahrens ergeben.

Nachfolgend wird die Erfindung beispielhaft anhand der beigefügten Figuren beschrieben. In diesen zeigt:
- Fig. 1: eine schematische Gesamtansicht eines medizinischen Instrumentes gemäß der Erfindung,
- Fig. 2: schematisch den abgewinkelten Endoskopkopf,
- Fig. 3: schematisch die Freiheitsgrade der Bewegung des Endoskopkopfes gemäß Figuren 1 und 2,
- Fig. 4: schematisch die Anordnung der Aktoren in dem Endoskopkopf gemäß Figuren 1 bis 3,
- Fig. 5: eine Draufsicht auf das distale Ende des Endoskopkopfes,
- Fig. 6: eine Schnittansicht entlang der Linie VI - VI in Fig. 5,
- Fig. 7: eine Schnittansicht entlang der Linie VII - VII in Fig. 5,
- Fig. 8: schematisch eine erste mögliche Elektrodeanordnung für einen Aktor
- Fig. 9: schematisch eine zweite mögliche Elektrodenanordnung für die Aktoren und
- Fig. 10: schematisch die Steuereinrichtung für ein erfindungsgemäßes Instrument.

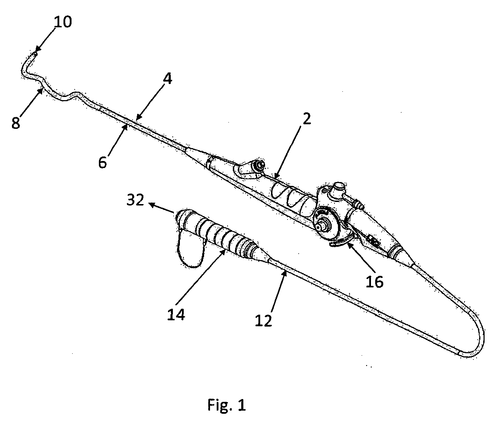

Wie Fig. 1 zeigt, weist das beispielhaft beschriebene Instrument einen am proximalen Ende angeordneten Handgriff 2 auf, von welchem sich ein Schaft 4 distalwärts erstreckt. Der Schaft 4 weist einen ersten proximalen Schaftabschnitt 6 auf, welcher flexibel ausgebildet ist. An das distale Ende des Schaftabschnittes 6 schließt sich ein aktiv beweglicher Endoskopkopf 8 an, dessen distales Ende die distale Endoskopspitze 10 bildet. Proximalseitig erstreckt sich von dem Handgriff 2 ein Anschlusskabel 12, welches in einem Anschlussstecker 14 endet, mit welchem das Instrument mit einem Steuergerät und gegebenenfalls mit einer Lichtquelle verbunden werden kann. Der Handgriff weist Bedienelemente 16 auf, über welche die Auslenkung der Endoskopspitze am Endoskopkopf 8 eingestellt werden kann.

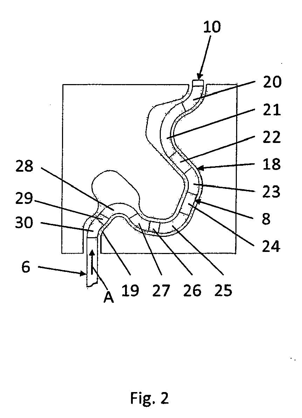

Wie in Fig. 1 zu erkennen ist, kann sich der Endoskopkopf 8 sehr flexibel verbiegen bzw. auslenken. Hierzu sind im Inneren des Endoskopkopfes 8, wie nachfolgend beschrieben werden wird, Aktoren angeordnet, welche die Auslenkung bewirken. Der Endoskopkopf 8 ermöglicht eine schlängelnde Endoskopbewegung, welche anhand von Fig. 2 näher erläutert wird.

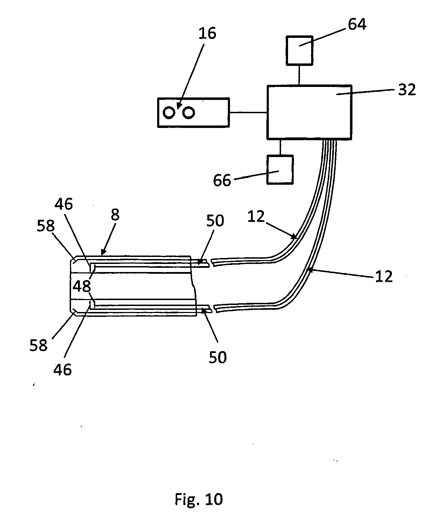

Fig. 2 zeigt den Endoskopkopf 8 in einem sich schlängelnden Kanal 18, beispielsweise einer geschlängelten Körperöffnung. Zum Einführen wird das distale Ende des Endoskopkopfes 8, d. h. die Endoskopspitze 10 in Richtung des Pfeils A in den Kanal 18 eingeschoben. Bei der ersten Biegung wird ein erster Abschnitt 20, welcher sich proximalseitig an die Endoskopspitze 10 anschließt, so gewinkelt bzw. gekrümmt, dass die erste Biegung 19 umrundet werden kann. Beim weiteren Vorschub in Richtung des Pfeils A wird der Abschnitt 20 jeweils entsprechend gelenkt oder gebogen, dass die folgenden Kurven umfahren werden können. Die sich proximalwärts an den Abschnitt 20 anschließenden Abschnitte 21 - 30 des Endoskopkopfes 8 können ebenfalls abgewinkelt werden, wobei die Abschnitte 21 - 30 beim weiteren Vorschub immer so abgewinkelt werden, dass sie der Bewegung des ersten Abschnittes 20 folgen, so dass der Endoskopkopf 8 sich in einer schlängelnden Bewegung durch den Kanal 18 bewegen kann. Dabei erfolgt die Auslenkung der Abschnitte 20 bis 30 aktiv durch in den Abschnitten angeordnete Aktoren. Dies hat den Vorteil, dass die Reibung an den Wandungen des Kanals 18 reduziert wird, da dort wesentlich geringere Druckkräfte auftreten. Somit ist insgesamt ein leichter Vorschub des Instrumentes möglich, wodurch sich die Verletzungsgefahr verringert. Die Auslenkung der Abschnitte 21 bis 30 wird vorzugsweise von einer Steuereinrichtung 32 (siehe Fig. 10) veranlasst. Die Steuereinrichtung steuert in Abhängigkeit des Vorschubes in Richtung A die Auslenkung der Abschnitte 21 bis 30 automatisch, so dass diese Bereiche den Bewegungen des ersten Abschnittes 20 nachgeführt werden, und sich insgesamt die gezeigte geschlängelte Bewegung ergibt.

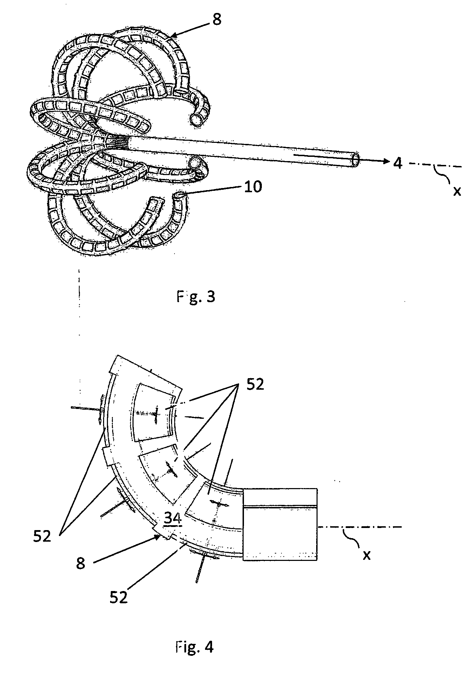

Fig. 3 zeigt schematisch, wie sich der Endoskopkopf 8 im Wesentlichen in jede beliebige radiale Richtung bezüglich der Längsachse X biegen bzw. auslenken kann. Dabei ist eine Biegung um jeweils etwa 270° möglich, so dass die Endoskopspitze 10 in radialer Richtung der Längsachse X des Schaftes 4 zugewandt ist. So zeigt Fig. 3 die maximal mögliche Auslenkung und die möglichen Auslenkungsrichtungen des Endoskopkopfes 8.

Die Auslenkung des Endoskopkopfes 8 wird durch in dem Endoskopkopf 8 angeordnete Aktoren ermöglicht. Dies wird näher anhand der Figuren 4 bis 9 erläutert. Der Endoskopkopf 8 ist als ein integriertes Bauteil ausgebildet, welches mehrere Funktionen vereint. Die Umfangswandung 34 sowie die stirnseitige Wandung 36 an der Endoskopspitze 10 sind einstückig aus einem Polymermaterial gefertigt. Bei diesem Polymermaterial handelt es sich um ein elektroaktives Polymer, d. h. ein Polymer, welches in Abhängigkeit einer angelegten elektrischen Spannung seine Form bzw. Ausdehnung ändert. In die stirnseitige Wandung 36 sind die distalseitigen Schnittstellen des Instrumentes integriert. Im gezeigten Beispiel handelt es sich dabei um einen Sondenkanal 38, eine Optik 40, sowie um Lichtleiter 42 und 44. Alle diese Kanäle und Elemente enden in der Endoskopspitze 10 und weisen dort ihre distalseitigen Schnittstellen auf. In entsprechender Weise könnte hier Bildsensoren, LED-Lichtquellen und ähnliches als distalseitige Schnittstellen in die stirnseitige Wandung 36 integriert werden. Die stirnseitige Wandung 36 und die Umfangswandung 34 bilden die mechanisch tragende Struktur des Endoskopkopfes 8. Das heißt, die Umfangswandung 34 bildet in diesem Bereich das Rohr des Schaftes, welches den Schaft auch gleichzeitig nach außen begrenzt.

In das elektroaktive Polymer sind Elektroden 46 und 48 eingebettet. Die Elektroden 46 bilden Steuerelektroden, während die Elektroden 48 Referenzelektroden darstellen. Zwischen den Elektroden 46 und 48, welche abwechselnd beabstandet zueinander in dem Polymer 34 angeordnet sind, wird eine Spannung angelegt, wodurch sich die Ausdehnung des Polymers zwischen den Elektroden 46 und 48 verändert. Dadurch können Längenänderung in Richtung der Längsachse X hervorgerufen werden. Da die Elektroden 46 und 48 in Elektrodengruppen angeordnet sind, welche an verschiedenen Stellen des Umfangs in der Umfangswandung 34 angeordnet sind, können diese Längenänderungen durch entsprechende elektrischen Spannungen an den Elektroden in einzelnen Umfangsbereichen selektiv hervorgerufen werden, wodurch eine Auslenkung bzw. Ausbiegung in der zuvor beschriebenen Weise erzielt wird. Die Elektroden 46 und 48 sind über Steuerleitungen 50 mit der Steuereinrichtung 32 verbunden. Die Steuerleitungen 50 sind ebenfalls in das Polymer der Wandung 34 eingebettet und erstrecken sich in proximaler Richtung bis in den Handgriff 2, von wo aus sie dann elektrisch leitend weiter mit der Steuereinrichtung 32 verbunden sind.

Dadurch, dass die Elektroden 46 und 48 direkt in der Umfangswandung 34 angeordnet sind, können die Aktoren, welche aus den Elektroden 46 und 48 sowie dem zwischen den Elektroden gelegen Polymer, d. h. dem Material der Wandung 34, gebildet werden, sehr platzsparend in dem Instrument angeordnet werden. Es wird kein zusätzlicher Bauraum für die Aktoren benötigt, diese können in die Wandung 34 integriert werden bzw. die Wandung selber wirkt als Aktor. Dadurch kann das freie Lumen im Inneren für die Kanäle, Lichtleiter und ähnliches sehr groß gehalten werden. Darüber hinaus ist der Aufbau des Instrumentes sehr einfach, da der Endoskopkopf einstückig ausgebildet ist. Die Endoskopspitze 10 kann gemeinsam mit der Umfangswandung 34 aus dem elektroaktiven Polymer gegossen werden, wobei gleichzeitig die für die Aktoren erforderlichen Elektroden 46 und 48 mit ihren Steuerleitungen 50 in das Polymer eingegossen werden. Das heißt, es entfällt eine zusätzliche Montage für die Aktoren und die Endoskopspitze 10.

Da der Abstand zwischen den einzelnen Elektroden 46 und 48 sehr klein ist, ist auch die erzielbare Längenänderung zwischen den einzelnen Elektroden 46 und 48 sehr klein. Aus diesem Grunde werden mehrere Elektroden zu Elektrodengruppen zusammengefasst. Dabei werden die Elektroden einer Elektrodengruppe dann vorzugsweise gemeinsam mit Spannung beaufschlagt, so dass sich die einzelnen Längenänderungen zwischen den einzelnen Elektroden im Polymer insgesamt zu einer größeren Längenänderung addieren. Diese Elektrodengruppen bilden Aktorfelder 52, welche schematisch in Fig. 4 gezeigt sind. In Fig. 4 sind die Elektroden nicht gezeigt, sondern lediglich die Bereiche (Aktorfelder 52), in welchen die Elektroden in die Umfangswandung 34 eingebettet sind. Dabei werden die Elektroden, wie vorangehend beschrieben vorzugsweise in die Umfangswandung eingegossen, so dass eine Umfangswandung entsteht, welche einstückig aus Polymermaterial gefertigt ist.

Die Aktorfelder 52 sind zum einen so angeordnet, dass in Umfangsrichtung der Wandung 34 mehrere Aktorfelder 52 verteilt sind. Das heißt, in dieser Richtung sind die Aktorfelder 52 an verschiedenen Umfangspositionen, vorzugsweise gleichmäßig verteilt gelegen. Hierdurch kann die Auslenkung in eine bestimmte Richtung, wie in Fig. 3 schematisch gezeigt, erreicht werden. Je nach dem welches der Aktorfelder 52, in einer Querschnittsebene mit Spannung beaufschlagt wird, wird der Schaft in entsprechender Richtung ausgelenkt. In Fig. 4 gezeigten Beispiels sind drei Aktorfelder 52 über den Umfang verteilt. Wenn nur diese drei Aktorfelder 52 selektiv mit Spannung beaufschlagt würden, wäre eine Auslenkung nur in drei radiale Richtungen möglich. Es ist jedoch auch möglich, zwei der Aktorfelder 52 gleichzeitig mit Spannung zu beaufschlagen, so dass auch eine Auslenkung in eine Winkelrichtung, welche zwischen zwei Aktorfelder 52 gelegen ist, möglich ist. Darüber hinaus ist es durch Variation der elektrischen Spannungen, mit welcher die Elektroden der einzelnen Aktorfelder 52 beaufschlagt werden, auch möglich, den Grad der Formänderung zwischen den Elektroden und somit die Auslenkung zu variieren. Wenn nun zwei Aktorfelder 52 in derselben Querschnittsebene mit unterschiedlichen Spannungen beaufschlagt werden, kann durch entsprechende Wahl der Spannungen somit eine Auslenkung in jede beliebige Richtung erreicht werden, wie in Fig. 3 gezeigt ist.

Darüber hinaus sind die Aktorfelder 52 auch in axialer Richtung hintereinander liegend angeordnet. Dadurch werden in axialer Richtung die Abschnitte 20 bis 30 geschaffen, welche unterschiedlich ausgelenkt werden können, wodurch die schlangenförmige Biegbarkeit des Instrumentes realisiert wird, wie anhand von Fig. 2 erläutert.

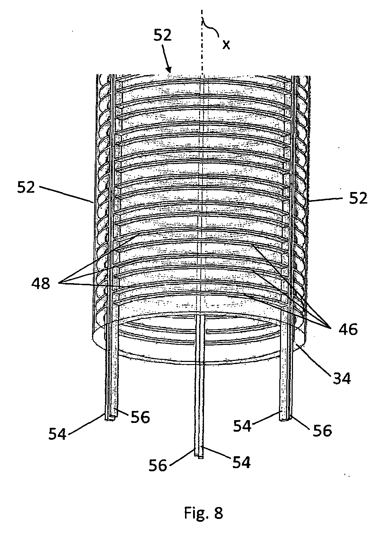

Ein erstes Beispiel für eine mögliche Anordnung der Elektroden in den Aktorfeldern 52 ist in Fig. 8 gezeigt. Fig. 8 zeigt in einer Umfangsebene drei Aktorfelder 52, welche durch abwechselnd angeordnete Elektroden 46 und 48 gebildet werden. Bei dem Ausführungsbeispiel gemäß Fig. 8 ist eine kammförmige Elektrodenstruktur gewählt. Das heißt, die Elektroden 46 erstrecken sich ausgehend von axialen Leitern 54 in Umfangsrichtung jeweils im Wesentlichen über einen Drittelkreis. Entsprechend erstrecken sich die Elektroden 48 ausgehend von axialen Leitern 56 jeweils ebenfalls in Umfangsrichtung im Wesentlichen über einen Drittelkreis. Dabei erstrecken sich die Elektroden 46 und 48 ausgehend von den axialen Leitern 54 und 56 in entgegengesetzte Richtungen, so dass die Elektroden 46 in die Freiräume zwischen den Elektroden 48 eingreifen bzw. umgekehrt die Elektroden 48 in die Freiräume zwischen den Elektroden 46 eingreifen. Da alle Elektroden 46 eines Aktorfeldes 52 mit dem zugehörigen axialen Leiter 54 verbunden sind, werden diese Elektroden 46 alle gemeinsam mit Spannung gegenüber den Elektroden 48 beaufschlagt. Die Elektroden 48 eines Aktorfeldes 52 sind jeweils gemeinsam mit einem axialen Leiter 56 verbunden. Die axialen Leiter 54 und 56 erstrecken sich in axialer Richtung zum proximalen Ende des Schaftes als Steuerleitungen 50.

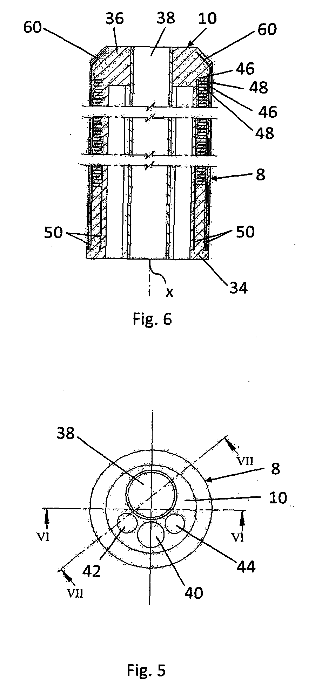

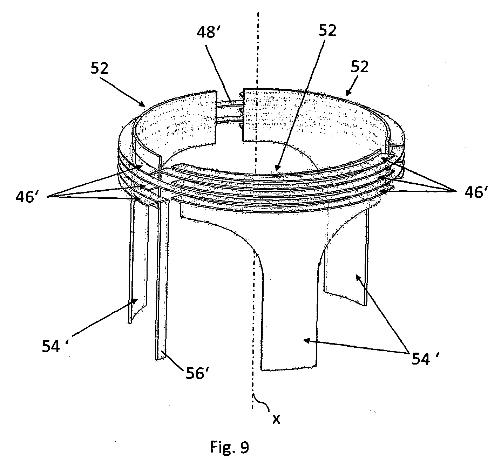

Fig. 9 zeigt eine zweite mögliche Elektrodenanordnung. Bei der Anordnung gemäß Fig. 9 ist die Referenzelektrode 48' als durchgehende wendel- bzw. schraubenförmige Referenzelektrode 48' ausgebildet. An einem Ende ist diese Elektrode 48' mit einem axialen Leiter 56' verbunden, welcher in proximaler Richtung eine Steuerleitung 50 bildet. Auch bei der Ausführungsform gemäß Fig. 9 sind drei in Umfangsrichtung verteilt angeordnete Aktorfelder 52 vorgesehen, welche sich im Wesentlichen jeweils über einen Drittelkreis erstrecken. Die Steuerelektroden 46' sind jeweils segmentförmig ausgebildet und erstrecken sich ebenfalls im Wesentlich über einen Drittelkreis. Dabei sind in jedem Aktorfeld 52 zahlreiche bogenförmige Steuerelektroden 46' so beabstandet voneinander angeordnet, dass sie in die Freiräume zwischen den Wendelgängen der durchgehenden Referenzelektrode 48' eingreifen. Die bogenförmigen Steuerelektroden 46' erstrecken sich ausgehend von axialen Leitern 54' in radialer Richtung stegförmig nach Außen. So werden drei Gruppen von Steuerelektroden 46' geschaffen, welche jeweils über einen der axialen Leiter 54' mit Spannung gegenüber der Referenzelektrode 48' beaufschlagt werden können. Auf diese Weise werden drei unabhängig voneinander betätigbare Aktorfelder 52 geschaffen. Die axialen Leiter 54' erstrecken sich in proximaler Richtung als Steuerleitungen 50 weiter. Bei der Ausführungsform gemäß Fig. 9 ist zu verstehen, dass die dort gezeigte Elektrodenanordnung wie in den Figuren 6, 7 und 8 gezeigt in die Umfangswandung 34 des Endoskopkopfes 8 eingegossen ist. Wenn die Elektroden 46', 48' in die Umfangswandung 34 eingegossen sind, befindet sich zwischen den Elektroden 46' und 48' elektroaktives Polymermaterial, welches ein Dialektrikum bildet und unter einer zwischen den Elektroden 46' und 48' angelegten Spannung seine Ausdehnung in axialer Richtung X verändert.

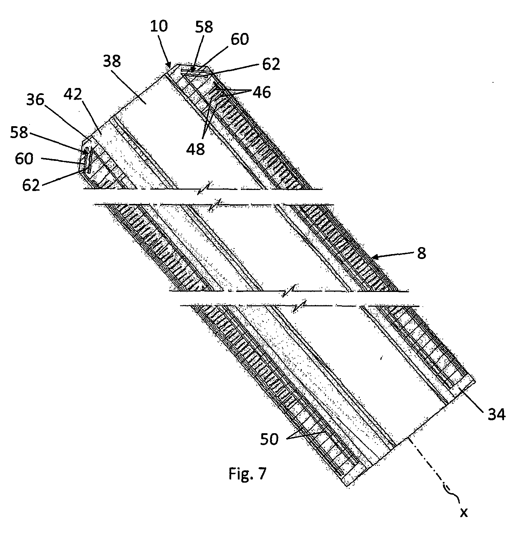

In der Endoskopspitze 10 sind an mehreren Umfangspositionen, wie in Fig. 7 gezeigt, Kraftsensoren 58 angeordnet. Die Kraftsensoren 58 sind jeweils aus zwei in das Polymermaterial der Wandungen 34 und 36 eingebettete und zueinander beabstandete Elektroden 60 und 62 gebildet. Die Elektroden 60 und 62 sind in axialer Richtung mit Steverleitungen 50 verbunden, welche sich zum proximalen Ende des Instrumentes erstrecken und dort dann über das Anschlusskabel 12 mit der Steuereinrichtung 32 verbunden werden. Die Kraftsensoren 58 arbeiten in der Weise, das bei Verformung des Polymermateriales zwischen den Elektroden 60 und 62 dieses seine Kapazität und/oder seinen elektrischen Widerstand ändert, was durch die Elektroden 60 und 62 erfasst werden kann. Dazu sind die Elektroden 60 und 62 über Steuerleitungen 50 mit der Steuereinrichtung 32 verbunden, welche den Widerstand bzw. die Kapazität zwischen den Elektroden 60 und 62 erfasst. In Kenntnis des Elastizitätsmoduls des Polymers der Wandung 34 und 36 kann dann in Abhängigkeit der erfassten Kapazität oder des elektrischen Widerstandes auf die einwirkende Kraft geschlossen werden. Dadurch, dass die Elektroden 60 und 62 geneigt zu der Längsachse X und der Stirnfläche der Endoskopspitze 10 angeordnet sind, hier in einem Winkel von im Wesentlichen 45°, können von den Kraftsensoren 58 Kräfte mit Komponenten aus axialer Richtung und radialer Richtung erfasst werden. Darüber hinaus sind mehrere Kraftsensoren 58 über den Umfang der Endoskopspitze 10 verteilt angeordnet. So kann bei selektiver Auswertung der Signale der einzelnen Kraftsensoren 58 von der Steuereinrichtung 32 erkannt werden, in welchem Umfangsbereich eine Kraft auf die Endoskopspitze 10 ausgeübt wird. Eine solche Kraft wird insbesondere beim Vorschub erzeugt, wenn die Endoskopspitze 10 gegen ein Hindernis stößt.

Wenn sich nun das Hindernis in nur einem Umfangsbereich befindet, kann das Endoskop durch Auslenkung des Endoskopkopfes 8, zunächst des Bereiches 20 (siehe Fig. 2), dieses Hindernis umfahren. Die Steuereinrichtung 32 ist dazu so ausgebildet, dass sie eine automatische Auslenkung ermöglicht, wenn die Endoskopspitze 10 auf ein Hindernis trifft. Dann wird von der Steuereinrichtung 32 erfasst, in welchem Umfangsbereich das Hindernis liegt, abhängig davon welcher der Kraftsensoren 58 eine erhöhte Kraft signalisiert. Anschließend werden die Elektroden 46 und 48 in denjenigen Aktorfelder 52 mit Spannung beaufschlagt, welche eine Auslenkung des Endoskopkopfes 8 in eine der erfassten Kraft abgewantdte bzw. entgegengesetzte radiale Richtung verursacht. Auf diese Weise wird die Endoskopspitze 10 von dem Hindernis in radialer Richtung weggelenkt bzw. weggebogen, so dass die Endoskopspitze 10 und beim weiteren Vorschub der Endoskopkopf 8 um das Hindernis herumgeführt werden kann.

Es erfolgt dabei zweckmäßigerweise eine kontinuierliche Kraftüberwachung an den Kraftsensoren 58 und eine umgehende Steuerung der zugehörigen Aktoren bzw. Elektroden 46 und 48 durch die Steuereinrichtung 32, so dass insgesamt die anhand von Fig. 2 beschriebene schlängelnde Bewegung des Endoskopkopfes 8 erzielt werden kann.

Wenn mehrere oder alle Kraftsensoren 58 eine erhöhte Kraft signalisieren, lässt dies auf eine frontal in axialer Richtung X wirkende Kraft schließen. Dies bedeutet eine frontale Kollision mit einem Hindernis. In einem solchen Fall ist es der Steuereinrichtung 32 nicht ohne weiteres möglich, zu erkennen, in welche Richtung der Endoskopkopf 8 ausgelenkt werden muss, um das Hindernis zu passieren. In diesem Fall kann dann von der Steuereinrichtung 32 ein Alarmsignal über eine Alarmeinrichtung 64 ausgegeben werden, welche einer Bedienperson das Hindernis signalisiert. Die Alarmeinrichtung 64 kann als optische Anzeige, als akustische Anzeige, als akustischer Melder oder auch als habtischer Melder ausgebildet sein. Der haptische Melder kann beispielsweise ein Vibrieren im Handgriff oder ähnliches erzeugen. Ferner ist es auch möglich, dass die Steuereinrichtung 32 den Vorschub automatisch steuert. In einem solchen Fall kann bei einer frontalen Kollision der Vorschub gestoppt werden.

Um die sich schlängelnde Bewegung, welche anhand von Fig. 2 beschrieben wurde, zu erzeugen, muss die Steuereinrichtung 32 ferner Informationen über den zurückgelegten Vorschubweg erhalten. Hierzu kann eine Erfassungseinrichtung 66 zum Erfassen des zurückgelegten Vorschubweges vorgesehen werden. Diese kann beispielsweise in einem festgelegten Punkt angeordnet sein, dem gegenüber die relative axiale Bewegung des Schaftes 4 erfasst wird. Abhängig von der Ausdehnung bzw. Stauchung des Polymers zwischen den Elektroden 46, 46' und 48, 48' ändert sich der elektrische Widerstand des Polymers, was über die Elektroden 46, 46' und 48, 48' erfasst werden kann.

### Bezugszeichenliste

- 2: - Handgriff
- 4: - Schaft
- 6: - Schaftabschnitt
- 8: - Endoskopkopf
- 10: - Endoskopspitze
- 12: - Anschlusskabel
- 14: - Anschlussstecker
- 16: - Bedienelemente
- 18: - Kanal
- 19: - Biegung
- 20 - 30: - Abschnitte des Endoskopkopfes
- 32: - Steuereinrichtung
- 34: - Umfangswandung
- 36: - stirnseitige Wandung
- 38: - Sondenkanal
- 40: - Optik
- 42: - Lichtleiter
- 44: - Lichtleiter
- 46, 46', 48, 48': - Elektroden
- 50: - Steuerleitungen
- 52: - Aktorfelder
- 54, 54', 56,56': - axiale Leiter
- 58: - Kraftsensoren
- 60,62: - Elektroden
- 64: - Alarmeinrichtung
- 66: - Erfassungseinrichtung
- X: - Längsachse
- A: - Vorschubrichtung

## Patentansprüche

1. Endoskopisches Instrument mit einem in zumindest einem Abschnitt biegbaren Schaft (4) und einem das distale Ende des Schaftes (4) bildenden Endoskopkopf (8), in welchem die distalseitigen Schnittstellen des Instrumentes angeordnet sind, wobei
der Endoskopkopf (8) biegbar ist und seine Umfangswandung (34) sowie seine stirnseitige Wandung (36) einstückig aus einem elektroaktiven Polymer ausgebildet sind, wobei in dem Endoskopkopf (8) in dem Polymer Elektroden (46, 48) angeordnet sind, welche mit einer elektrischen Spannung beaufschlagbar sind.

2. Endoskopisches Instrument nach Anspruch 1, **dadurch gekennzeichnet, dass** die Elektroden (46. 48) in das Polymer eingegossen sind.

3. Endoskopisches Instrument nach Anspruch 1 oder 2, **dadurch gekennzeichnet, dass** die Elektroden (46,48) zumindest eine Elektrodengruppe bestehend aus zumindest einer Steuerelektrode (46) und zumindest einer Referenzelektrode (48) aufweisen.

4. Endoskopisches Instrument nach Anspruch 3, **dadurch gekennzeichnet, dass** der Endoskopkopf (3) mehrere Elektrodengruppen (52) aufweist, welche an verschiedenen Winkelpositionen bezüglich der Längsachse X des Instrumentes angeordnet sind.

5. Endoskopisches Instrument nach Anspruch 3 oder 4, **dadurch gekennzeichnet, dass** der Endoskopkopf (8) mehrere Elektrodengruppen (52) aufweist, welche an verschiedenen axialen Positionen bezüglich der Längsachse X des Instrumentes angeordnet sind.

6. Endoskopisches Instrument nach einem der Ansprüche 3 bis 5, **dadurch gekennzeichnet, dass** zumindest eine Elektrodengruppe (52) vorgesehen ist, welche mehrere in axialer Richtung X bezüglich der Längsachse des Instrumentes voneinander beabstandete Steuerelektroden (46) aufweist, welche vorzugsweise unabhängig voneinander mit Spannung beaufschlagbar sind.

7. Endoskopisches Instrument nach Anspruch 6, **dadurch gekennzeichnet, dass** die Elektrodengruppe (52) eine Vielzahl von Steuerelektroden (46) und eine Vielzahl von Referenzelektroden (48) aufweist, welche in axialer Richtung X einander abwechselnd angeordnet sind.

8. Endoskopisches Instrument nach Anspruch 7, **dadurch gekennzeichnet, dass** die Vielzahl von Steuerelektroden (46) und die Vielzahl von Referenzelektroden (48) jeweils als Kammstruktur ausgebildet sind und die beiden Kammstrukturen mit ihren Zähnen ineinander greifen.

9. Endoskopisches Instrument nach Anspruch 6, **dadurch gekennzeichnet, dass** die Referenzelektrode (48') schraubenförmig ausgebildet ist und Steuerelektroden (46') zwischen einzelnen Schraubengängen der Referenzelektrode (48') angeordnet sind.

10. Endoskopisches Instrument nach einem der Ansprüche 3 bis 10, **dadurch gekennzeichnet, dass** eine Mehrzahl von Elektrodengruppen (52) eine gemeinsame Referenzelektrode(48), insbesondere in schraubenförmige Gestalt, aufweist.

11. Endoskopisches Instrument nach einem der vorangehenden Ansprüche, **dadurch gekennzeichnet, dass** der Schaft (4) proximalseitig des Endoskopkopfes (8) zumindest einen weiteren biegbaren Abschnitt aus einem elektroaktiven Polymer mit darin angeordneten Elektroden aufweist.

12. Endoskopisches Instrument nach einem der vorangehenden Ansprüche, **dadurch gekennzeichnet, dass** die Elektroden (46. 48) mit Steuerleitungen (50) verbunden sind, welche sich zum proximalen Ende des Schaftes (4) erstrecken.

13. Endoskopisches Instrument nach einem der vorangehenden Ansprüche, **dadurch gekennzeichnet, dass** der Endoskopkopf (8) zumindest einen am distalen Ende angeordneten Kraftsensor (58) aufweist.

14. Endoskopisches Instrument nach Anspruch 13, **dadurch gekennzeichnet, dass** der Kraftsensor (58) in das elektroaktive Polymer eingebettet ist.

15. Endoskopisches Instrument nach einem der vorangehenden Ansprüche, **dadurch gekennzeichnet, dass** es eine elektronische Steuereinrichtung (32) aufweist, welche mit den Elektroden (46, 48) verbunden ist und die Verformung des Schaftes (4) steuert.

## Claims

1. An endoscopic instrument with a shank (4) which is bendable in at least one section and with an endoscope head (8) which forms the distal end of the shank (4) and in which distal-side interfaces of the instrument are arranged, wherein the endoscope head (8) is bendable and its peripheral wall (34) as well as its face-side wall (34) are designed as one piece from an electroactive polymer, wherein electrodes (46, 48) are arranged in the endoscope head (8) in the polymer and can be subjected to an electrical voltage.

2. An endoscopic instrument according to claim 1, **characterised in that** the electrodes (46, 48) are moulded into the polymer.

3. An endoscopic instrument according to claim 1 or 2, **characterised in that** the electrodes (46, 48) comprise at least one electrode group consisting of at least one control electrode (46) and at least one reference electrode (48).

4. An endoscopic instrument according to claim 3, **characterised in that** the endoscope head (3) comprises several electrode groups (52), which are arranged at different angular positions with respect to a longitudinal axis X of the instrument.

5. An endoscopic instrument according to claim 3 or 4, **characterised in that** the endoscope head (8) comprises several electrode groups (52), which are arranged at different axial positions with regard to a longitudinal axis X of the instrument.

6. An endoscopic instrument according to one of the claims 3 to 5, **characterised in that** at least one electrode group (52) is provided, which comprises several control electrodes (46) spaced from one another in an axial direction X with respect to the longitudinal axis of the instrument and able to be subjected to voltage, preferably independently of one another.

7. An endoscopic instrument according to claim 6, **characterised in that** the electrode group (52) comprises a multitude of control electrodes (46) and a multitude of reference electrodes (48), which are arranged alternating with one another in the axial direction X.

8. An endoscopic instrument according to claim 7, **characterised in that** the multitude of control electrodes (46) and the multitude of reference electrodes (48) are designed in each case as a comb structure, and the two comb structures mesh into one another with their teeth.

9. An endoscopic instrument according to claim 6, **characterised in that** the reference electrode (48') is designed in a helical manner and the control electrodes (46') are arranged between individual helix flights of the reference electrode (48').

10. An endoscopic instrument according to one of the claims 3 to 10, **characterised in that** a plurality of electrode groups (52) have a common reference electrode (48), in particular in a helical form.

11. An endoscopic instrument according to one of the preceding claims, **characterised in that** the shank (4) on the proximal side of the endoscope head (8) comprises at least one further bendable section of an electroactive polymer with electrodes arranged therein.

12. An endoscopic instrument according to one of the preceding claims, **characterised in that** the electrodes (46, 48) are connected to control leads (50), which extend to the proximal end of the shank (4).

13. An endoscopic instrument according to one of the preceding claims, **characterised in that** the endoscope head (8) comprises at least one force sensor (58) arranged at a distal end.

14. An endoscopic instrument according to claim 13, **characterised in that** the force sensor (58) is embedded into the electroactive polymer.

15. An endoscopic instrument according to one of the preceding claims, **characterised in that** it comprises an electronic control device (32), which is connected to the electrodes (46, 48) and controls the deformation of the shank (4).

## Revendications

1. Instrument endoscopique comprenant une tige (4) dont au moins une partie est flexible et une tête d'endoscope (8) formant l'extrémité distale de la tige (4), dans laquelle sont disposées les interfaces côté distal de l'instrument, dans lequel
la tête d'endoscope (8) est flexible et sa paroi périphérique (34) ainsi que sa paroi avant (36) sont réalisées d'un seul tenant à partir d'un polymère électroactif, des électrodes (46, 48) auxquelles peut être appliquée une tension électrique étant disposées dans la tête d'endoscope (8), dans le polymère.

2. Instrument endoscopique selon la revendication 1, **caractérisé en ce que** les électrodes (46, 48) sont noyées dans le polymère.

3. Instrument endoscopique selon la revendication 1 ou 2, **caractérisé en ce que** les électrodes (46, 48) présentent au moins un groupe d'électrodes constitué d'au moins une électrode de commande (46) et d'au moins une électrode de référence (48).

4. Instrument endoscopique selon la revendication 3, **caractérisé en ce que** la tête d'endoscope (3) présente plusieurs groupes d'électrodes (52) qui sont disposés selon différentes positions angulaires par rapport à l'axe longitudinal X de l'instrument.

5. Instrument endoscopique selon la revendication 3 ou 4, **caractérisé en ce que** la tête d'endoscope (8) présente plusieurs groupes d'électrodes (52) qui sont disposés selon différentes positions axiales par rapport à l'axe longitudinal X de l'instrument.

6. Instrument endoscopique selon l'une des revendications 3 à 5, **caractérisé en ce qu'**est prévu au moins un groupe d'électrodes (52) qui présente plusieurs électrodes de commande (46), espacées les unes des autres dans la direction axiale X par rapport à l'axe longitudinal de l'instrument, auxquelles une tension peut être appliquée, de préférence indépendamment les unes des autres.

7. instrument endoscopique selon la revendication 6, **caractérisé en ce que** le groupe d'électrodes (52) présente une pluralité d'électrodes de commande (46) et une pluralité d'électrodes de référence (48) qui sont disposées en alternance dans la direction axiale X.

8. Instrument endoscopique selon la revendication 7, **caractérisé en ce que** la pluralité d'électrodes de commande (46) et la pluralité d'électrodes de référence (48) sont respectivement conçues sous la forme d'une structure formant peigne et **en ce que** les deux structures formant peigne viennent mutuellement en prise par leurs dents.

9. Instrument endoscopique selon la revendication 6, **caractérisé en ce que** l'électrode de référence (48') est réalisée sous forme hélicoïdale et **en ce que** des électrodes de commande (46') sont disposées entre les différentes spires de l'électrode de référence (48').

10. Instrument endoscopique selon l'une des revendications 3 à 10, **caractérisé en ce qu'**une pluralité de groupes d'électrodes (52) présente une électrode de référence commune (48), en particulier de forme hélicoïdale.

11. Instrument endoscopique selon l'une des revendications précédentes, **caractérisé en ce que** la tige (4) présente, côté proximal de la tête d'endoscope (8), au moins une autre partie flexible en polymère électroactif dans lequel sont disposées des électrodes.

12. Instrument endoscopique selon l'une des revendications précédentes, **caractérisé en ce que** les électrodes (46, 48) sont reliées à des conduits de commande (50) qui s'étendent jusqu'à l'extrémité proximale de la tige (4).

13. Instrument endoscopique selon l'une des revendications précédentes, **caractérisé en ce que** la tête d'endoscope (8) présente au moins un capteur de force (58) disposé à l'extrémité distale.

14. Instrument endoscopique selon la revendication 13, **caractérisé en ce que** le capteur de force (58) est noyé dans le polymère. électroactif.

15. Instrument endoscopique selon l'une des revendications précédentes, **caractérisé en ce qu'**il présente un dispositif de commande électronique (32) qui est relié aux électrodes (46, 48) et commande la déformation de la tige (4).
